**Europäisches Patentamt**

**European Patent Office**

**Office européen des brevets**

(19)

(11) Veröffentlichungsnummer: **0 355 582 B1**

# EUROPÄISCHE PATENTSCHRIFT

(12)

(45) Veröffentlichungstag der Patentschrift: **02.12.92**

(51) Int. Cl.5: **C07K 1/04**, B01J 19/00, B01L 3/02, G01N 35/06

(21) Anmeldenummer: **89114710.0**

(22) Anmeldetag: **09.08.89**

(54) **Verfahren und Vorrichtung zur vollautomatischen simultanen Synthese mehrerer Polypeptide.**

(30) Priorität: **23.08.88 DE 3828576**

(43) Veröffentlichungstag der Anmeldung:
**28.02.90 Patentblatt 90/09**

(45) Bekanntmachung des Hinweises auf die Patenterteilung:
**02.12.92 Patentblatt 92/49**

(84) Benannte Vertragsstaaten:
**AT BE CH DE ES FR GB GR IT LI LU NL SE**

(56) Entgegenhaltungen:
US-A- 3 985 032
US-A- 4 059 020

CHEMICAL ABSTRACTS, Band 106, Nr. 19, 11. Mai 1987, Seite 729, Zusammenfassung Nr. 156848a, Columbus, Ohio, US; I. BLAHA et al.: "Automatic device for solid-phase peptide synthesis"

(73) Patentinhaber: **BOEHRINGER INGELHEIM KG**
**Postfach 200**
**W-6507 Ingelheim am Rhein(DE)**

(84) Benannte Vertragsstaaten:
**BE CH DE ES FR GR IT LI LU NL SE AT**

(73) Patentinhaber: **BOEHRINGER INGELHEIM IN-TERNATIONAL GmbH**
**Postfach 20**
**W-6507 Ingelheim am Rhein(DE)**

(84) Benannte Vertragsstaaten:
**GB**

(72) Erfinder: **Schnorrenberg, Gerd, Dr.**
**Ernst-Ludwig-Strasse 66a**
**W-6535 Gau-Algesheim(DE)**
Erfinder: **Knapp, Wilhelm**
**Winzerstrasse 5**
**W-6537 Gensingen(DE)**

EP 0 355 582 B1

## Beschreibung

Für die schnelle Evaluierung von Struktur-Wirkungsbeziehungen an biologisch aktiven Peptiden durch Rezeptor-Bindungs-Studien und die schnelle Epitop-Ermittlung für die Immunologie bei Peptiden und Proteinen werden relativ kleine Mengen (unter je 20 mg) einer Vielzahl von Peptiden benötigt. Die Herstellung dieser Peptide erfolgt zweckmäßig nach der Festphasenpeptidsynthese. Diese Synthese basiert auf der von R.B. Merrifield entwickelten Methode (G. Barany, R.B. Merrifield in The Peptides, Analysis, Synthesis, Biology, Vol. 2, 3-284 (1980), Hrsg, Gross, Meienhofer Academic Press, New York), bei der die Peptidkette schrittweise aufgebaut wird. Die Syntheseschritte können wie folgt zusammengefaßt werden:

a) Binden der ersten Aminosäure der Peptidkette über eine Ankergruppe an einen polymeren Träger,

b) schrittweises Ankondensieren der übrigen Aminosäuren der Peptidkette,

c) Zwischenschritte zwischen den einzelnen Kondensationen bestehend aus Waschen, Abspalten von Schutzgruppen und Neutralisieren,

d) gewünschtenfalls acylieren endständiger Aminogruppen,

e) Abspalten des Peptids vom Träger.

Bei dieser Peptidsynthese muß mit einer Synthesezeit von bis zu 18 Stunden, meist bis zu 4 Stunden pro Aminosäure gerechnet werden. (Die einzelnen Kondensationen benötigen meist 1 bis 2 Stunden Reaktionszeit; zwischen den Kondensationen sind in der Regel etwa 10 Zwischenschritte erforderlich, für die je ca. 2 bis 15 Minuten gerechnet werden müssen.) Die Herstellung von Peptiden bestehend aus einer größeren Anzahl von Aminosäuren ist somit sehr langwierig, arbeitsintensiv und teuer.

Für die Festphasensynthese analoger Peptide ist von R. A. Houghten (Proc. Natl. Acad. Sci, USA, Vol. 82, pp. 5131-5135 August 1985, Immunology) eine Methode beschrieben worden. Danach wird der polymere Träger für die Synthese in Portionen von je 50-100 mg in kleine Poröse Polypropylenbeutel gefüllt, die Beutel werden zugeschmolzen, die in den Synthesen einheitlichen Zwischenschritte (Waschen, Neutralisieren, Abspalten von Schutzgruppen) werden an allen Beuteln gleichzeitig in einem gemeinsamen Reaktionsgefäß ausgeführt die einzelnen Kondensationen werden getrennt ausgeführt. Die Methode kann manuell oder teilweise automatisiert unter Verwendung eines Peptidsynthesizers ausgeführt werden.

Der Nachteil der beschriebenen Methode liegt darin, daß die Handhabung der Beutel etwas umständlich ist, daß die Beutel nicht wieder verwendet werden können, daß für die Kondensationen der verschiedenen Peptide die Beutel voneinander getrennt werden müssen und keine Kontrollproben während der ganzen Synthese entnommen werden können.

Der Erfindung liegt die Aufgabe zugrunde, ein Verfahren und eine Vorrichtung bereit zu stellen, die die automatische simultane Synthese mehrerer Polypeptide ermöglicht und die oben genannten Nachteile vermeidet.

Die Aufgabe wird gelöst, indem die bereits erwähnte Festphasensynthese-Methode so abgewandelt wird, daß sie mit Hilfe eines entsprechend angepaßten Pipettier-Roboters ausgeführt werden kann. Pipettier-Roboter sind bis jetzt für Serienanalysen verwendet worden. Zum Beispiel ist ein Pipettier-Roboter der Firma TECAN, RSP 5052, verwendbar.

Pipettier-Roboter weisen folgende äußere Bestandteile auf: Mindestens einen Arm mit Dosierpipette, eine Halterung mit Vorratsgefäßen sowie eine Mikrotiterplatte die bis zu 96 Wells enthalten kann. Der Arm des Roboters bringt die Reagenzien aus den Vorratsgefäßen in die jeweiligen Wells der Microtiterplatte ein und saugt bei Bedarf Flüssigkeiten aus den Wells ab. Die Kanüle der Dosierpipette kann auch so ausgebildet sein, daß sie durch eine von oben nach unten laufende Trennwand in zwei Teile geteilt ist. (Durch diese geteilte Kanüle ist es möglich, zwei verschiedene Zudosierungen oder eine Zudosierung und eine Absaugung mit einem Arm auszuführen) Der Arbeitsablauf des Gerätes wird durch ein Computerprogramm gesteuert.

Die Festphasenpeptidsynthese erfolgt erfindungsgemäß in einem solchen Pipettier-Roboter wie folgt:
In den Wells einer Microtiterplatte wird Trägermaterial (vorzugsweise granuliertes Trägermaterial) vorgelegt. Das Trägermaterial kann mit dem Anfangsteil des jeweils gewünschten Peptids beladen sein. Die für die Reaktionen und Waschschritte benötigten Flüssigkeiten werden in den Vorratsgefäßen des Gerätes bereitgestellt. Soll am Ende der Synthese das Peptid vom Träger getrennt werden und/oder sollen freie Aminogruppen acyliert werden, so sind auch für diese Reaktionen die erforderlichen Reagenzien in den Vorratsgefäßen vorzubereiten. Aus den benötigten Reaktionszeiten ergibt sich, daß es zweckmäßig ist, eine Microtiterplatte zu verwenden, die nicht mehr als 96 Wells enthält. Dementsprechend können in einem Programmablauf maximal 96 verschiedene Polypeptide synthetisiert werden. Entsprechend dem Programm, das der Synthese dieser Peptide angepaßt worden ist, bringt der Roboter die Reagenzien und Waschflüssigkeiten in die einzelnen Wells ein und saugt nach der entsprechenden Verweilzeit die über dem Träger stehende Flüssigkeit jeweils ab.

Anhand des zweiarmigen Pipettier-Roboters RSP 5052 der Firme TECAN wird das Verfahren und die nötige Anpassung des Gerätes an das Verfahren näher erläutert. Die Anwendung des Verfahrens ist jedoch nicht auf dieses Gerät beschränkt. Pipettier-Roboter anderer Bauart, insbesondere auch ein- oder mehrarmige Roboter können gemäß der vorliegenden Erfindung für das Verfahren angepaßt werden.

Es wird eine Microtiterplatte mit 96 Wells gewählt. Ein Well faßt z. B. 10 mg Harz, das mit einer Aminosaure beladen sein kann, und etwas mehr als 300 $\mu$l Flüssigkeit. Diese Menge Harz entspricht etwa 5 $\mu$mol Aminosäure bzw. ist für die Herstellung von etwa 5 $\mu$mol Peptid geeignet. Übliche Trägermaterialien auf Polystyrol- oder Polyacrylamidbasis sind verwendbar. Es ist zweckmäßig, Peptide aufzubauen, die maximal 20 Aminosäuren enthalten. Die dafür benötigten Reagenzlösungen und Waschflüssigkeiten werden in den dafür vorgesehenen Vorratsgefäßen vorbereitet. Arm 1 des Gerätes ist mit einer Dosierpipette versehen. Arm 2 mit einer Absaugkanüle mit Spülvorrichtung. Diese Spülvorrichtung ist vorzugsweise mit einem separat stehenden Vorratsgefäß für das verwendete Lösungsmittel verbunden. Die Synthese erfolgt nach dem im angeschlossenen PC vorgegebenen Programm.

Mit Arm 1 erfolgt die Zudosierung sämtlicher Reagenzlösungen, die aus offenen Vorratsgefäßen entnommen werden. Bevor die Dosierpipette von einer Reagenzlösung zu einer anderen wechselt, wird die Dosierpipette in einer speziellen Spülposition mit Lösungsmittel gespült. Mit Arm 2 erfolgt über eine mit einem Filter versehene Kanüle das Absaugen der Reagenz-und Waschflüssigkeiten. Zur Verhinderung von Harzverlusten und Kontaminationen der benachbarten Wells wird die Außenseite dieser Kanüle nach jedem Absaugvorgang mit Lösungsmittel über eine an der Kanüle seitlich angebrachte Zuleitung gespült. Mit diesem Lösungsmittel wird gleichzeitig der nächste Waschvorgang begonnen. Anschließend wird die Kanüle in einer Kanülenspülposition abgespült.

Für die Abtrennung des Peptids von Harz wird z.B. Trifluoressigsäure über Arm 1 in die Wells eingebracht.

Nach erfolgter Abspaltung wird mit der Absaugkanüle die Lösung abgesaugt und in eine zweite Mikrotiterplatte überführt, aus der dann die Aufarbeitung erfolgt.

Wie oben erwähnt worden ist, kann die Absaugkanüle mit einem Filter versehen sein. Dieses Filter ist am unteren Ende der Kanüle angebracht und verhindert das Absaugen und Mitreißen von Harz. Zweckmäßig kann das Filter aus einem Edelstahldrahtnetz bestehen, das z.B. mit einem Lötring befestigt wird. Eine andere Möglichkeit besteht darin, eine Metallsinterplatte oder Keramiksinterplatte in die Öffnung der Kanüle einzupassen. Wird eine Kanüle ohne Filter verwendet, so kann durch langsames Absaugen das Mitreißen und Absaugen von Harz weitgehend vermieden werden. Der Zeitbedarf für das Absaugen steigt dadurch jedoch erheblich.

Die Außenseite der Absaugkanüle wird nach jedem Absaugvorgang mit Lösungsmittel gespült. Das erfolgt zweckmäßig durch die Zufuhr des Lösungsmittels in einer Zuleitung (Schlauch oder Rohr), die an der Langsseite der Kanüle verläuft und so angebracht ist, daß die Öffnung knapp oberhalb des unteren Endes der Kanüle liegt. Es ist wichtig, daß das Lösungsmittel den ganzen Umfang des unteren Endes der Kanüle umspült.

Arm 2 des Gerätes kann mit einem Kamm anstelle der Absaugkanüle versehen werden. Ein solcher Kamm faßt mehrere Absaugkanülen (meist 4-12 Kanülen) der oben beschriebenen Ausführungsart zusammen. Mit Hilfe eines solchen Kammes können gleichzeitig mehrere Wells bedient werden, was zu einer wesentlichen Zeitersparnis führt.

Im allgemeinen wird DMF oder N-Methylpyrrolidon als Lösungsmittel eingesetzt. Dementsprechend müssen die Wells und die eventuell vorgesehene Spülvorrichtung aus lösungsmittelbestandigem Material gefertigt sein, z.B. aus Polypropylen oder Teflon. In den handelsüblichen Geräten sind die Dosierpipetten und Absaugkanülen aus Edelstahl gefertigt. Dieses Material ist für das erfindungsgemäße Verfahren geeignet.

Wenn man bei dieser Synthese Pro Peptid eine größere Harzmenge einsetzen will (z.B. 50 mg Harz) müssen Microtiterplatten mit größeren Wells verwendet werden. Diese Microtiterplatten sind genormt und im Handel erhältlich. Sie enthalten dann entsprechend weniger Wells.

Statt eines Zweiarm-Pipettier-Roboters kann auch ein Einarm-Gerät verwendet werden. In diesem Fall muß die Absaugkanüle eine Unterteilung im freien Volumen besitzen. Durch den einen Teil erfolgt die Absaugung der Wasch- und Reagenzlösungen, der andere Teil der Kanüle erfüllt die Funktion des Armes 1 des Zweiarm-Geräts, d.h. dient der Zudoserung der Reagenzlösungen. Der Schutz gegen Absaugen von Harz erfolgt wie beim Zweiarm-Gerät beschrieben. Auch diese Kanüle wird wie oben beschrieben abgespült.

Das Verfahren wird zum Beispiel mit folgenden Mitteln ausgeführt (Mengenangabe pro Well): Ausgangsmaterial ist 10 mg mit Fmoc-Aminosäuren beladenes Harz (Korngröße 200-400 mesh); Fmoc geschützte Aminosäuren werden in bis zu 10 fachem Überschuß für jeden einzelnen Kupplungsschritt eingesetzt, d.h. 200 $\mu$l einer DMF-Lösung von 50 $\mu$mol Fmoc-Aminosäure und 50 $\mu$mol 1-Hydroxybenzotriazol und 100 $\mu$l einer DMF-Lösung

von 75 $\mu$mol N,N-Dicyclohexylcarbodiimid werden zugegeben; die Kupplungszeit beträgt ca. 1 Stunde. Die Abspaltung der Fmoc-Schutzgruppe erfolgt jeweils mittels 300 $\mu$l einer 40%igen Lösung von Piperidin in DMF. Die Abspaltungszeit ist ca. 20 Minuten. Die Waschschritte werden mit jeweils 300$\mu$l DMF ausgeführt.

Die Abspaltung des fertigen Peptids vom Träger kann in den Wells erfolgen durch manuelle oder automatische Zugabe von 300 $\mu$l Trifluoressigsäure (20 Minuten Reaktionszeit). Das Acylieren von freien Gruppen (NH$_2$, OH) kann analog durch Zugabe geeigneter Säureanhydride, z.B. Acetanhydrid und Pyridin, erfolgen. Nach Beendigung dieser Umsetzungen wird die Lösung abgesaugt und der Aufarbeitung zugeführt.

Wie aus der obigen Erläuterung deutlich wird, werden alle Schritte der Peptidsynthese in offenen Gefäßen ausgeführt. Durch die erfindungsgemäße Ausführung der Synthese werden die Peptide trotzdem in sehr hoher Reinheit erhalten.

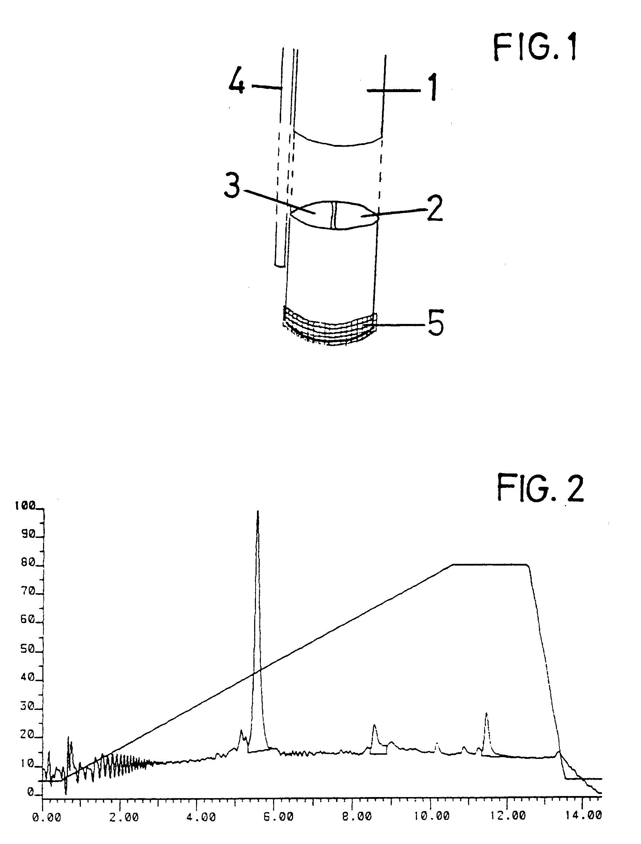

Figur 1 zeigt ein Beispiel einer Kanüle für einen Einarm-Pipettier-Roboter: (1) Kanüle; (2) und (3) Zudosierseite bzw. Absaugseite der Kanüle; (4) Schlauch für Zufuhr des Lösungsmittels zur Spülung; (5) Filter.

Das folgende Beispiel zeigt den Ablauf des Verfahrens, wobei der Aufbau eines Peptides in einem Well beschrieben wird. Zu beachten ist die Reinheit des Produktes.

Beispiel:

Es wurden 44 verschiedene Undekapeptide mit Hilfe des Tecan-Pipettier-Roboters RSP 5052 synthetisiert. Ausgangsmaterial war jeweils ein über einen Linker an Polystyrol gebundenes Tetrapeptid Fmoc-Arg(Mtr)-Gln-Arg(Mtr)-Tyr(tBu)-Linker-Polystyrol (Beladung ca. 0.5 mmol/g Harz). 44 mal wurden jeweils 10 mg dieses Harzes in die Wells einer Mikrotiterplatte (5 $\mu$mol Peptid) gebracht und die Synthese begonnen. Als Beispiel wird die Synthese von

Ac-His-Tyr-Ile-Asn-Leu-Ile-Thr-Arg-Gln-Arg-Tyr-NH$_2$

beschrieben. Die Synthese der übrigen 44 Peptide verlief analog.

Synthesecyclus:

300 $\mu$l Piperidin (40%) in DMF (5 min)
300 $\mu$l Piperidin (40%) in DMF (20 min)
10 mal Waschen mit je 300 $\mu$l DMF (2 min)
200 $\mu$l einer DMF-Löung 50 $\mu$molar an Fmoc-Thr-(tBu)-OH und 50 $\mu$molar an HOBt
100 $\mu$l einer DMF-Lösung 75 $\mu$molar an DCC (1h)
10 mal Waschen mit je 300 $\mu$l DMF (2 min)

Entsprechend wurden gleichkonzentrierte Lösungen von

Fmoc-Ile-OH
Fmoc-Leu-OH
Fmoc-Asn-OH
Fmoc-Ile-OH
Fmoc-Tyr(tBu)-OH
Fmoc-His(Trt)-OH

in identischen Synthesecyclen verwendet. Die abschließende Acetylierung wurde nach Fmoc-Spaltung wie üblich mit 300 $\mu$l einer DMF-Lösung 40 $\mu$molar an Acetanhydrid und Pyridin durchgeführt.
Die Abspaltung vom Harz erfolgte mit 300 $\mu$l Trifluoressigsäure/1% Anisol 30min, waschen. 5 mal mit je 300 $\mu$l Trifluoressigsäure. Die vereinigten Trifluoressigsäurelösungen wurden unter Feuchtigkeitsausschluß 1 h bei 50°C stehengelassen und die Trifluoressigsäure im Vakuum abdestilliert. Der Rückstand wurde mit Ether im Ultraschallbad behandelt, Ether dekantiert, der Rückstand mit wenig Wasser aufgenommen und gefriergetrocknet. HPLC-Chromatogramm (RP18-Säule) (Fig. 2).

Laufmittel A     Wasser/Acetonitril/Trifluoressigsäure 95/5/0.2
Laufmittel B     Wasser/Acetonitril/Trifluoressigsäure 20/80/0.2

FAB-Massenspektrogramm: M + H : 1518

**Patentansprüche**

1. Verfahren zur vollautomatischen simultanen Synthese mehrerer Polypeptide nach der Festphasensynthesemethode unter Benutzung eines Pipettier-Roboters, wonach polymeres Trägermaterial, das mit dem Anfangsteil des jeweils gewünschten Peptids beladen sein kann, in den Wells einer Microtiterplatte vorgelegt wird, dann nach der an sich bekannten Festphasensynthesemethode in den Wells die Peptide aufgebaut werden und gewünschtenfalls freie Aminogruppen und/oder Hydroxygruppen der Peptide acyliert werden und/oder anschließend die Peptide von ihrem Trägermaterial abgetrennt werden, indem die für die einzelnen Schritte erforderlichen Reagenzien beziehungsweise Waschflüssigkeiten durch einen oder mehrere Roboterarm(e) mit Kanüle aus den entsprechenden Vorratsbehältern in die Wells eingebracht werden und jeweils nach der benötigten Verweilzeit der Reagenzien beziehungsweise Waschflüssigkeiten die in den Wells oberhalb des Trägermaterials befindlichen Flüssigkeiten durch einen Roboterarm mit Kanüle abgesaugt werden, wobei die einzelnen Schritte des Verfahrens durch das im Computer, der an den Roboter angeschlossen ist, vorgegebene Programm gesteuert werden.

2. Verfahren nach Anspruch 1, bei dem jeweils zwischen dem Absaugen der Flüssigkeit und dem Zugeben von Reagenzien die Außenseite der Kanüle gespült wird.

3. Verfahren nach Anspruch 1 oder 2, wobei die Reagenzien in bis zu 20 fachem Überschuß der stöchiometrisch benötigten Menge eingesetzt werden.

4. Kanüle für einen Pipettier-Roboter, deren oberes Ende an die Dosiervorrichtung des Roboterarmes angeschlossen ist, die mit einer Spülvorrichtung versehen ist, deren oberes Ende an dem Roboterarm angeschlossen ist und deren unteres Ende bis knapp oberhalb des unteren Endes der Kanüle reicht, deren Öffnung so angeordnet ist, daß das untere Ende der Kanüle gespült werden kann.

5. Kanüle nach Anspruch 4, deren unteres Ende mit einem Filter versehen ist.

6. Kanüle nach Anspruch 4 oder 5, deren Hohlraum durch eine von oben nach unten laufende Trennwand in 2 Teile geteilt ist.

7. Kanülenkamm, bestehend aus mehreren Kanülen nach einem der Ansprüche 4 bis 6.

8. Verwendung eines Pipettier-Roboters zur vollautomatischen simultanen Synthese mehrerer Polypeptide nach der Festphasensynthesemethode.

**Claims**

1. Process for fully automatic simultaneous synthesis of a plurality of polypeptides by the solid phase synthesis method using a pipetting robot, according to which polymeric carrier material, which may be charged with the initial portion of the desired peptide, is placed in the wells of a microtitre plate, then using the solid phase synthesis method which is known per se the peptides are synthesised in the wells and if desired free amino groups and/or hydroxy groups of the peptides are acylated and/or subsequently the peptides are separated from their carrier material, the reagents or washing liquids required for the individual steps being introduced into the wells by means of one or more robot arms with a cannula from the corresponding storage vessels and after the required retention time of the reagents or washing liquids the liquids contained in the wells above the carrier material are sucked up by means of a robot arm with a cannula, the individual steps of the process being controlled by the program in the computer which is connected to the robot.

2. Process according to claim 1, in which the outside of the cannula is rinsed between the sucking up of the liquid and the addition of the reagents.

3. Process according to claim 1 or 2, in which the reagents are used in an excess of up to 20 times the stoichiometric amount.

4. Cannula for a pipetting robot, the upper end of which is connected to the metering device of the robot arm, which is provided with rinsing means, the upper end of which is connected to the robot arm whilst the lower end thereof extends to just above the lower end of the cannula, the opening of which is arranged so that the lower end of the cannula can be rinsed.

5. Cannula according to claim 4, the lower end of which is equipped with a filter.

6. Cannula according to claim 4 or 5, the cavity of which is subdivided into two parts by a partition wall extending from top to bottom.

7. Cannula comb, consisting of a plurality of cannulae according to one of claims 4 to 6.

8. Use of a pipetting robot for the fully automatic simultaneous synthesis of a plurality of polypeptides by the solid phase synthesis method.

**Revendications**

1. Procédé de synthèse simultanée totalement automatisée de plusieurs polypeptides selon le procédé de synthèse en phase solide au moyen d'un robot de pipettage, selon lequel un matériau support polymérique, qui peut être chargé par la portion initiale du peptide souhaité dans chaque cas, est disposé au préalable dans les puits d'une plaque de microtitrage, puis les peptides sont formés dans les puits selon le procédé de synthèse en phase solide connu en soi et, si on le souhaite, des groupes amine et/ou hydroxyle libres des peptides sont acylés et/ou les peptides sont ensuite séparés de leur matériau support, les réactifs ou liquides de lavage nécessaires pour les différentes étapes étant introduits dans les puits depuis les récipients de stockage correspondants par un ou plusieurs bras de robot munis d'une canule et, à chaque fois, le temps de séjour nécessaire des réactifs ou liquides de lavage étant écoulé, les liquides qui se trouvent dans les puits au dessus du matériau support sont aspirés par un bras de robot muni d'une canule, les différentes étapes du procédé étant commandées par le programme pré-établi dans l'ordinateur qui est relié au robot.

2. Procédé selon la revendication 1, dans lequel à chaque fois entre l'aspiration du liquide et l'addition des réactifs le côté extérieur de la canule est lavé.

3. Procédé selon la revendication 1 ou 2, dans lequel les réactifs sont utilisés en un excès atteignant 20 fois la quantité nécessaire du point de vue stoechiométrique.

4. Canule pour robot de pipettage, dont l'extrémité supérieure est reliée au dispositif doseur du bras de robot, qui est muni d'un dispositif de lavage dont l'extrémité supérieure est reliée au bras de robot et dont l'extrémité inférieure arrive juste au dessus de l'extrémité inférieure de la canule dont l'ouverture est agencée de telle manière que l'extrémité inférieure de la canule peut être lavée.

5. Canule selon la revendication 4, dont l'extrémité inférieure est munie d'un filtre.

6. Canule selon la revendication 4 ou 5, dont la cavité est divisée en deux parties par une paroi de séparation qui s'étend de haut en bas.

7. Peigne de canules consistant en plusieurs canules selon l'une des revendications 4 à 6.

8. Utilisation d'un robot de pipettage pour la synthèse simultanée totalement automatisée de plusieurs polypeptides selon le procédé de synthèse en phase solide.

# FIG.1

# FIG. 2